Europäisches Patentamt

European Patent Office

Office européen des brevets

(11) Numéro de publication: **0 305 278**
**A1**

(12) # DEMANDE DE BREVET EUROPEEN

(21) Numéro de dépôt: 88402125.4

(22) Date de dépôt: 18.08.88

(51) Int. Cl.⁴: **C 12 N 5/00**

(30) Priorité: 20.08.87 FR 8711776

(43) Date de publication de la demande:
01.03.89 Bulletin 89/09

(84) Etats contractants désignés:
AT BE CH DE ES GB GR IT LI LU NL SE

(71) Demandeur: BIOLOGIE ET INDUSTRIE
30 boulevard Thiers
F-52000 Chaumont (FR)

(72) Inventeur: Roseto, Alberto
31, Boulevard Guy de Maupassant
F-78400 Chatou (FR)

(74) Mandataire: Peaucelle, Chantal et al
S.C. Ernest Gutmann - Yves Plasseraud 67, boulevard Haussmann
F-75008 Paris (FR)

(54) Adjuvants pour la production et la culture d'hybridomes.

(57) L'adjuvant de l'invention est formé par un épanchement dans un espace liquidien d un organisme animal, tel qu'obtenu par greffe, dans une paroi délimitant cet espace, de l'une des lignées cellulaires utilisées pour l'obtention de l'hybridome à cultiver.

**Description**

## ADJUVANTS POUR LA PRODUCTION ET LA CULTURE D'HYBRIDOMES

L'invention a pour objet des adjuvants pour la production et la culture d'hybridomes.

On sait que la culture d'hybridomes est utilisée pour produire des anticorps monoclonaux.

La technique classique d'hybridation lymphocytaire comprend la fusion, en présence d'un agent fusogène tel que le polyéthylène glycol, de cellules spléniques d'une souris préalablement immunisée avec un antigène, avec des cellules myelomateuses.

L'utilisation d'un milieu sélectif permet d'éliminer les cellules non hybrides. Les hybridomes producteurs des anticorps monoclonaux, les facteurs recherchés, sont clonés et sélectionnés à partir d une technique de criblage immunologique appropriée et sont mis en culture.

Des milieux de culture particulièrement utilisés sont élaborés à partir de sérums, plus spécialement du sérum de veau foetal, qui est très largement mis en oeuvre pour la culture de toute cellule.

On connaît, cependant, l'intérêt de disposer pour ce type de culture de sérums de grande spécificité, adaptés à ces hybridomes.

Les travaux des Inventeurs dans ce domaine les ont conduits à constater que certains épanchements liquidiens peuvent être utilisés avec avantage comme adjuvants de milieux de culture d'hybridomes.

L'invention a donc pour but de fournir des adjuvants particulièrement adaptés à la culture des hybridomes, permettant d'améliorer le rendement dès l'étape de fusion et capables d'agir sur la multiplication cellulaire dès le premier stade de multiplication.

Ces adjuvants sont caractérisés en ce qu'il s'agit d'un épanchement, dans un espace liquidien d'un organisme animal, tel qu'obtenu par greffe, dans une paroi délimitant cet espace, de l'une des lignées cellulaires utilisées pour l'obtention de l'hybridome à cultiver.

Avantageusement, la lignée cellulaire utilisée pour la greffe est une lignée cellulaire myélomateuse de même espèce que celle utilisée pour l'obtention de l'hybridome à cultiver.

L'espace liquidien est constitué soit par l'espace péritonéal compris entre l'intestin et la paroi abdominale, soit par l'espace intra-pleural, situé entre les poumons et la paroi thoracique, ou encore par l'espace péricardique, compris entre le coeur et le sac péricardique qui l'enveloppe.

D'une manière avantageuse, la greffe effectuée produit, dans l'espace liquidien utilisé, une accumulation de liquide capable de favoriser dans un procédé de fusion et de culture, la fusion et la prolifération des hybridomes tout en assurant un taux de secrétion élevé d'immunoglobulines.

Les adjuvants de l'invention favorisent notamment la production d'anticorps monoclonaux d'origines diverses.

Ces anticorps sont produits par des cellules de rate d'animaux immunisés, sensibilisés à des antigènes viraux, bactériens, cellulaires (cellules entières d'origine humaine).

Selon un mode préféré de réalisation de l'invention, l'adjuvant est constitué par un liquide d'ascite.

Il s'agit d un adjuvant de grande stabilité, permettant d'améliorer la croissance, puis la secrétion des hybridomes en culture.

Ce liquide est ponctionné de la cavité organique, débarrassé, par exemple par centrifugation, des cellules et autres impuretés, telles qu agrégats moléculaires et autres particules solides en suspension, et conditionné en vue de son utilisation comme adjuvant.

D'autres caractéristiques et avantages de l'invention apparaîtront dans les exemples qui suivent.

Exemple 1 : Culture d'hybridomes de souris provenant de la fusion de lignées cellulaires SpO₂ de souris et de rate de souris immunisées :

On réalise la culture avec, à titre comparatif, un milieu classique (milieu 1) et, dans une autre expérience, avec un milieu selon l'invention (milieu 2). Les milieux présentent respectivement les compositions suivantes :
milieu 1 : RPMI (Roswell Park Medium Institute) + 1% glutamine + 0,3 % glucose + 1 % pyruvate de sodium + 7 % de sérum de veau foetal (SVF) décomplémenté (c'est-à-dire chauffé au bain-marie 1 heure à 55°C pour détruire les protéines du système complémentaire à réaction immunogène.
milieu 2 : sa composition est identique à celle du milieu 1 mais le sérum de veau décomplémenté est remplacé par du liquide d'ascite purifié obtenu par greffe dans le péritoine de souris de cellules SpO₂, cellules myélomateuses de souris.

On ensemence des boîtes de culture 1 et 2 avec 64.000 cellules et on ajoute dans chacune 6 ml soit de milieu 1, soit de milieu 2.

Les observations effectuées après 24, 48 et 72 heures de culture conduisent aux résultats suivants :

|                         | 24 heures  | 48 heures | 72 heures |
|-------------------------|------------|-----------|-----------|
| nombre de cellules dans boîte 1 : | 800.000 | 1.400.000 | 3.640.000 |
| nombre de cellules dans boîte 2 : | 1.200.000 | 2.300.000 | 4.000.000 |

Ces résultats mettent en évidence l'effet avantageux de l'adjuvant selon l'invention pour la culture de l'hybridome.

Exemple 2 :

On compare le nombre de clones obtenus avec une culture d'hybridome réalisée avec un milieu classique contenant du SVF, et une autre culture dans laquelle est mis en oeuvre l'adjuvant de l'invention de l'exemple 1.

On prépare des microplaques en ensemençant des cellules de rate de souris fusionnées à des cellules $SpO_2$ à raison de 100 µl par puit, d'une solution contenant en moyenne une cellule par puit. Le milieu de culture utilisé ne renferme ni SVF ni adjuvant selon l'invention.

On compte les cellules d'hybridomes et on dilue afin d'obtenir 10 cellules par microplaque. On répartit ensuite les cellules dans les microplaques avec les milieux à tester renfermant différentes concentrations de SVF ou d'adjuvant.

On rapporte ci-dessous le nombre de clones par microplaques :

|        | SVF | Adjuvant de l'invention |
|--------|-----|-------------------------|
| 1 %    | O   | 5                       |
| 2,5 %  | O   | 6                       |
| 5 %    | 1   | 6                       |

L'examen de ces résultats montre l'intérêt des adjuvants de l'invention. Pour obtenir 6 clones avec un milieu classique renfermant du SVF, il est en effet nécessaire d'ajouter 20 % de SVF au milieu de culture, alors qu'un tel résultat est obtenu avec l'invention en utilisant seulement 2,5 % d'adjuvant.

L'invention fournit donc des milieux de culture simples, économiques et aisés à commercialiser sous forme liquide ou lyophilisée. Ces milieux favorisent la croissance d'hybridomes d'origines variées (souris, rat, homme...) ainsi que leur production d'anticorps monoclonaux.

**Revendications**

1. Adjuvant pour la production et la culture d'hybridomes, caractérisé en ce qu'il s'agit d'un épanchement dans un espace liquidien d'un organisme animal, tel qu'obtenu par greffe, dans une paroi délimitant cet espace, de l'une des lignées cellulaires utilisées pour l'obtention de l'hybridome à cultiver.

2. Adjuvant selon la revendication 1, caractérisé en ce que la lignée cellulaire utilisée pour la greffe est une lignée cellulaire myélomateuse de même espèce que celle utilisée pour l'obtention de l'hybridome à cultiver.

3. Adjuvant selon la revendication 1 ou 2, caractérisé en ce que l'espace liquidien est constitué, soit par l'espace péritonéal compris entre l'intestin et la paroi abdominale, soit par l'espace intra-pleural, situé entre les poumons et la paroi thoracique, ou encore par l'espace péricardique, compris entre le coeur et le sac péricardique qui l'enveloppe.

4. Adjuvant selon l'une quelconque des revendications 1 à 3, caractérisé en ce qu'il s'agit d'un liquide d'ascite.

5. Adjuvant selon la revendication 4, caractérisé en ce que le liquide d'ascite est tel qu'obtenu par greffe dans le péritoine de souris, de cellules de lignée cellulaire de souris utilisée dans l'étape de fusion de l'hybridome, récupération du liquide formé, et purification.

6. Milieu de culture élaboré à partir d'un adjuvant selon l'une quelconque des revendications 1 à 5.

Office européen
des brevets

**RAPPORT DE RECHERCHE EUROPEENNE**

Numero de la demande

EP 88 40 2125

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | PREPARATIVE BIOCHEMISTRY, vol. 15, no. 4, 1985, pages 221-236, Marcel Dekker Inc.; R.C. FELDHOFF et al.: "Purification of transferrin and albumin from mouse ascites fluid" * En entier * | 1-6 | C 12 N 5/00 |
| X | BIOLOGICAL ABSTRACTS, vol. 81, no. 1, 1986, résumé no. 4261, Philadelphia, P.A., US; Y. WANG et al.: "Application of feeder cells in generating human-human hybridomas" & CHIN. J. MICROBIOL. IMMUNOL (BEIJING) 5(2) 128-130, 1985 * Résumé * | 1-6 | |
| A | EP-A-0 076 647 (HANA BIOLOGICS, INC.) * Page 6, ligne 21 - page 7, ligne 9; page 13, lignes 2-17 * | 1 | |
| A | THE JOURNAL OF EXPERIMENTAL MEDICINE, vol. 147, 1978, pages 923-933; N.N. ISCOVE et al.: "Complete replacement of serum by albumin, transferrin, and soybean lipid in cultures of lipopolysaccharide-reactive B lymphocytes" * Page 929, ligne 12 - page 932 * | 1 | DOMAINES TECHNIQUES RECHERCHES (Int. Cl.4) C 12 N |
| A | ANALYTICAL BIOCHEMISTRY, vol. 102, no. 2, 1980, pages 255-270, Academic Press, Inc.; D. BARNES et al.: "Review - Methods for growth of cultured cells in serum free medium" * Page 265, colonne 1, lignes 16-31 * | 1 | |

Le présent rapport a été établi pour toutes les revendications

| Lieu de la recherche | Date d'achèvement de la recherche | Examinateur |
|---|---|---|
| LA HAYE | 14-11-1988 | SKELLY J.M. |